(19) Europäisches Patentamt
European Patent Office
Office européen des brevets

(11) Numéro de publication: **0 187 631**
**B1**

(12) **FASCICULE DE BREVET EUROPEEN**

(45) Date de publication du fascicule du brevet: **05.09.90**

(21) Numéro de dépôt: **85870178.2**

(22) Date de dépôt: **11.12.85**

(51) Int. Cl.[5]: **C 07 D 471/04,** C 07 F 7/08 // (C07D471/04, 221:00, 209:00)

(54) **Procédé pour la préparation de dérivés de pyrrolo-pyridine et nouveaux dérivés obtenus.**

(30) Priorité: **12.12.84 FR 8419029**

(43) Date de publication de la demande:
**16.07.86 Bulletin 86/29**

(45) Mention de la délivrance du brevet:
**05.09.90 Bulletin 90/36**

(84) Etats contractants désignés:
**AT BE CH DE FR GB IT LI LU NL SE**

(56) Documents cités:

**TETRAHEDRON, vol. 39, no. 10, pages 1777-1781, Pergamon Press Ltd., Oxford, GB; E. BISAGNI et al.: "Lithiation de furo- et pyrrolo3,2-crpyridines substituées sur leur position 4"**

(73) Titulaire: **SANOFI**
**40, Avenue George V**
**F-75008 Paris (FR)**

(72) Inventeur: **Dormoy, Jean-Robert**
**Avenue du Thor, 9**
**F-04200 Sisteron (FR)**
Inventeur: **Heymes, Alain**
**Rue Frédéric Mistral, 5**
**F-04200 Sisteron (FR)**

(74) Mandataire: **Bressand, Georges**
**c/o CABINET LAVOIX 2 Place d'Estienne d'Orves**
**F-75441 Paris Cédex 09 (FR)**

**EP 0 187 631 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention se rapporte, d'une manière générale, à la préparation de dérivés de 1H-pyrrolo[3,2-c]pyridine.

Plus précisément, l'invention concerne un procédé pour la fixation d'une groupement électrophile en position 2 de la 1H-pyrrolo[3,2-c]pyridine N-protégée en position 1.

A l'heure actuelle, on n'a rapporté dans la littérature chimique, qu'un nombre relativement restreint de dérivés de 1H-pyrrolo[3,2-c]pyridine substitués en position 2. La préparation de tels composés, il est vrai, s'effectue généralement avec grandes difficultés. Par exemple, on a cité dans J. Med. Chem. 1972, vol. 15, No. 11 pp. 1168—1171, la préparation de phényl-2 et de (fluoro-2 phényl)-2 1H-pyrrolo[3,2-c]pyridine selon une réaction de cyclisation à haute température et moyennant des conditions fortement basiques.

Ces conditions opératoires sont loin d'être applicables pour la réalisation de toute substitution en position 2 de la 1H-pyrrolo[3,2-c]pyridine puisque les auteurs de cette publication sont parvenus à la conclusion que "la réaction décrite est inappropriée pour la synthèse de dérivés de 1H-pyrrolo[3,2-c]pyridine comportant des substituants fragiles".

La recherche d'un procédé permettant une meilleure accessibilité aux dérivés de 1H-pyrrolo[3,2-c] pyridine substitués en position 2 quel que soit le genre de substituant à fixer reste donc d'un intérêt incontestable.

L'extrapolation systématique à partir de procédés connus pour l'introduction de substituants au niveau de l'indole n'est cependant pas applicable à la 1H-pyrrolo[3,2-c]pyridine étant donné la différence bien connue de réactivité entre ces deux hétérocycles.

On sait, par exemple, que les réactions au niveau de l'indole se déroulant en milieu acide sont difficilement envisageables dans le cas de la 1H-pyrrolo[3,2-c]pyridine étant donné la protonation et par conséquent la désactivation liée à l'azote pyridinique.

De même, on a rapporté dans J. C. S. Perkin I pp. 138—141 (1979) des essais de synthèse du pyrido[4,3-b]indole et des pyrido[4,3-b]indolyl-6 ou 7-carboxylates d'éthyle par condensation de la 1H-pyrrolo[3,2-c]pyridine avec l'hexane-2,5 dione ou le dioxo-2,5 hexyl-3 carboxylate d'éthyle.

Bien que ce type de réaction soit connu et utilisé au niveau de l'indole, les essais ci-dessus à partir de 1H-pyrrolo[3,2-c]pyridine se sont soldés par des échecs probablement en raison de la plus faible réactivité du noyau pyrrolique dans le cas de la pyrrolo-pyridine.

De plus, on a signalé dans Tetrahedron, vol. 39, No. 10 pp. 1777—1781 (1983), des tentatives effectuées en vue de fixer un substituant directement en position 2 de la 1H-pyrrolo[3,2-c]pyridine.

On a extrapolé, à cet effet, une méthode largement utilisée dans le cas de l'indole de manière à faire réagir la méthyl-1 1H-pyrrolo[3,2-c]pyridine d'abord avec le tertiobutyl lithium à −70°C ensuite avec le N,N-diméthyl-formamide à même température.

Cependant, ces réactions n'ont pas conduit au composé souhaité mais à des mélanges complexes à partir desquels uniquement une petite portion du produit de départ a été isolé.

Dans le cadre de l'élaboration de la présente invention, on a également entrepris des essais de lithiation régiosélective de la 1H-pyrrolo[3,2-c]pyridine selon une méthode décrite pour l'indole.

Selon cette méthode, on effectue la lithiation de la position 2 de l'indole à partir de ce composé protégé en position 1 et ce, dans le tétrahydrofuranne, à 0°C et au moyen de diisopropylamidure de lithium [J. Org. Chem. *46*, pp. 2979—2981 (1981)].

Appliqué à la 1H-pyrrolo[3,2-c]pyridine dans les mêmes conditions opératoires, ce procédé fournit un mélange de nombreux produits notamment du produit de départ non consommé, de la 1H-pyrrolo[3,2-c]pyridine libre en position 1 et un composé résultant de l'addition du diisopropylamidure dee lithium sur le noyau pyridinique.

L'ensemble de ces résultats met donc suffisamment en évidence l'impossibilité d'extrapoler à la 1H-pyrrolo[3,2-c]pyridine, des procédés largement connus dans la série de l'indole.

On est maintenant parvenu, dans le cadre de la présente invention, à préparer des dérivés de 1H-pyrrolo[3,2-c]pyridine selon un procédé mettant en oeuvre la protection de la position 1 de la 1H-pyrrolo[3,2-c]pyridine, la lithiation de la position 2 du composé obtenu et la substitution du dérivé lithio-2 ainsi préparé par un groupement électrophile approprié.

Ainsi, on peut fixer, selon l'invention, un groupement électrophile en position 2, de la 1H-pyrrolo[3,2-c]pyridine N-protégée en position 1 selon un procédé consistant:

a) à faire réagir, à température ambiante, la 1H-pyrrolo[3,2-c]pyridine dans un solvant, tel que par exemple le dichlorométhane, au moyen d'un hydroxyde de métal alcalin, tel que l'hydroxyde de lithium, de sodium ou de potassium et en présence d'un catalyseur de transfert de phase tel que le sulfate acide de tétrabutylammonium puis, à une température comprise entre la température ambiante et 40°C, avec un halogénure de formule générale:

R—Hal

dans laquelle R représente un groupement protecteur labile et Hal représente un atome de chlore, de brome ou d'iode, de préférence le chlore, pour obtenir les dérivés N-protégés de 1H-pyrrolo[3,2-c]pyridine de formule générale:

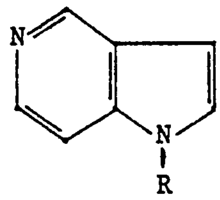

I

dans laquelle R a la même signification que précédemment,

b) à faire réagir dans un solvant tel qu'un éther par exemple le tétrahydrofuranne ou un mélange éther/hydrocarbure par exemple tétrahydrofuranne/pentane, le dérivé obtenu précédemment avec un agent de lithiation lequel est soit un amidure de lithium, soit un alkyl lithium, à une température comprise entre −80°C et −20°C et en présence de tétraméthyléthylènediamine pour obtenir les dérivés lithio-2 de formule générale:

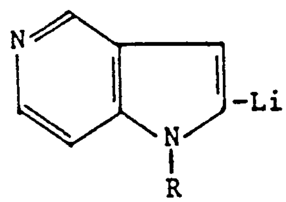

II

dans laquelle R a la même signification que précédemment,

c) puis à condenser le dérivé métallique obtenu, dans un solvant tel qu'un éther par exemple le tétrahydrofuranne ou un mélange éther/hydrocarbure par exemple tétrahydrofuranne/pentane et à une température comprise entre −80°C et la température ambiante, avec un réactif capable d'engendrer un groupement électrophile pour former des dérivés de 1H-pyrrolo[3,2-c]pyridine de formule I substitués en position 2 par un groupement électrophile.

Par groupement protecteur labile on entend un groupement méthoxyméthyle, un groupement benzyloxyméthyle, un groupement benzènesulfonyle ou p-toluènesulfonyle ou un groupement tertiobutoxycarbonyle (BOC).

On préfère en particulier le groupement benzènesulfonyle ou p-toluènesulfonyle ou le groupement BOC.

Ainsi le procédé de l'invention permet notamment la préparation des dérivés de la 1H-pyrrolo[3,2-c]pyridine de formule générale:

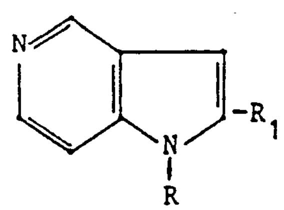

III

dans laquelle:

R représente un groupement protecteur labile tel que défini précédemment

$R_1$ représente:

un groupement alkyle inférieur

un groupement de formule:

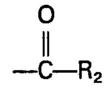

(A)

dans lequel $R_2$ représente hydrogène, un groupement alkyle inférieur, un groupement $-OR_3$ dans lequel $R_3$ représente hydrogène ou un groupement alkyle inférieur ou encore $R_2$ représente un groupement $-N(R_4)_2$ dans lequel $R_4$ représente un groupement alkyle inférieur,

un groupement de formule:

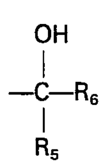

(B)

dans lequel $R_5$ représente hydrogène ou un groupement alkyle inférieur et $R_6$ représente phényle ou un groupement alkyle inférieur,

un groupement silyle de formule:

$$Si(R_6)_3 \qquad (C)$$

dans laquelle $R_6$ a la même signification que précédemment, procédé suivant lequel on condense un dérivé métallique de formule II dans un solvant tel qu'un éther par exemple le tétrahydrofuranne ou un mélange éther/hydrocarbure par exemple tétrahydrofuranne/pentane et à une température ambiante, avec un réactif capable d'engendrer un groupement électrophile choisi parmi le groupe formé par:

un halogénure d'alkyle de formule générale:

$$R_4—Hal$$

dans laquelle $R_4$ a la même signification que précédemment et Hal représente un atome de chlore, de brome ou d'iode, de préférence d'iode, pour obtenir les composés de formule III dans laquelle $R_1$ représente un groupement alkyle inférieur,

un ester de formule générale:

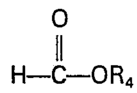

dans laquelle $R_4$ a la même signification que précédemment, ou un anhydride de formule générale:

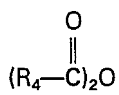

dans laquelle $R_4$ a la même signification que précédemment, ou l'anhydride carbonique, ou un halogénure de formule générale:

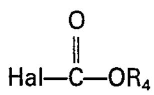

dans laquelle $R_4$ et Hal ont la même signification que précédemment, ou un halogénure de formule générale:

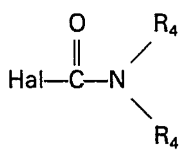

dans laquelle $R_4$ et Hal ont la même signification que précédemment, pour obtenir les composés de formule III dans laquelle $R_1$ représente un groupement de formule (A),

un aldéhyde ou une cétone de formule générale:

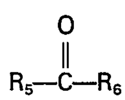

dans laquelle $R_5$ et $R_6$ ont la même signification que précédemment, pour obtenir les composés de formule III dans laquelle $R_1$ représente un groupement de formule (B),

un halogénure de silyle de formule générale:

$$Hal—Si(R_6)_3$$

dans laquelle Hal et $R_6$ ont la même signification précédemment pour obtenir les composés de formule III dans laquelle $R_1$ représente un groupement de formule (C). Par "groupement alkyle inférieur", on désigne plus spécialement les groupements méthyle, éthyle, n-propyle et isopropyle.

Les dérivés de 1H-apyrrolo[3,2-c]pyridine de formules I, II et III se sont révélés utiles comme intermédiaires de synthèse.

En conséquence, un autre objet de l'invention se rapporte aux composés de formules I, II et III en tant que produits industriels nouveaux utiles comme intermédiaires de synthèse, ces composés pouvant être représentés globalement par la formule générale:

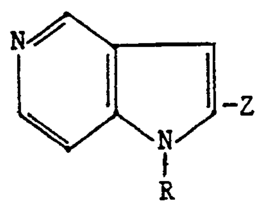

dans laquelle:

R représente un groupement protecteur labile tel que défini précédemment et,

Z représente un atome d'hydrogène, un atome de lithium ou l'un des groupements définis pour $R_1$ ci-dessus.

Comme cité ci-dessus, la préparation des dérivés N-protégés de formule II s'effectue notamment au départ d'un halogénure. Il est possible toutefois d'envisager l'emploi d'autres réactifs. Par exemple dans le cas où R représente un groupement tertiobutoxycarbonyle le dicarbonate de tertiobutyle sera préférentiellement utilisé.

L'alkyl lithium, utilisé dans le procédé de l'invention, peut être le tertiobutyl lithium et l'amidure de lithium est généralement le tétraméthyl-2,2,6,6 pipéridide de lithium, l'hexaméthyldisilylamidure de lithium ou, de préférence, le diisopropylamidure de lithium.

Ce dernier amidure de lithium constitue, en outre, l'agent de lithiation préféré selon l'invention.

Par équivalent de composés de formule I, on utilise de 1 à 2 équivalents d'agent de lithiation généralement une quantité de l'ordre de 1,8 équivalent et de 2,4 à 3 équivalents de réactif capable d'engendrer un groupement électrophile.

La tétraméthyléthylènediamine a essentiellement pour but de stabiliser, par ligandage, les dérivés lithio-2 de formule II.

En effet, on a remarqué que l'absence de tétraméthyléthylénediamine ou encore l'utilisation de températures plus élevées que celles envisagées dans le procédé de l'invention, entraînent une chute des rendements. Généralement, on utilise de 1 à 6 équivalents de tétraméthyléthylènediamine par équivalent de composé de formule I.

Ainsi, la métallation des composés de formule I pour l'obtention des dérivés lithiens de formule II peut s'effectuer selon différentes mises en oeuvre des réactifs, à savoir:

preparation extemporanée de l'amidure de lithium par réaction de butyllithium avec l'amine correspondante par exemple la diisopropylamine, la tétraméthylpipéridine ou l'hexaméthyldisilylamine et addition de cet amidure lithien à une solution du composé de formule I et de tétraméthyléthylènediamine,

préparation extemporanée de l'amidure de lithium par réaction de butyllithium avec l'amine correspondante en présence de tétraméthyléthylènediamine et addition de cette solution d'amidure lithien/tétraméthyléthylènediamine à une solution du composé de formule I,

addition de tertiobutyllithium à une solution du composés de formule I et de tétraméthyléthylènediamine,

addition de butyllithium à un mélange d'amine, de tétraméthyléthylènediamine (TMEDA) et de composé de formule I, telle que par exemple addition de 1,5 équivalent de butyllithium à un mélange de 0,5 équivalent d'amine, 1,5 équivalent de TMEDA et 1 équivalent de composé de formule I de manière à former in situ l'amidure de lithium et obtenir le schéma réactionnel ci-dessous:

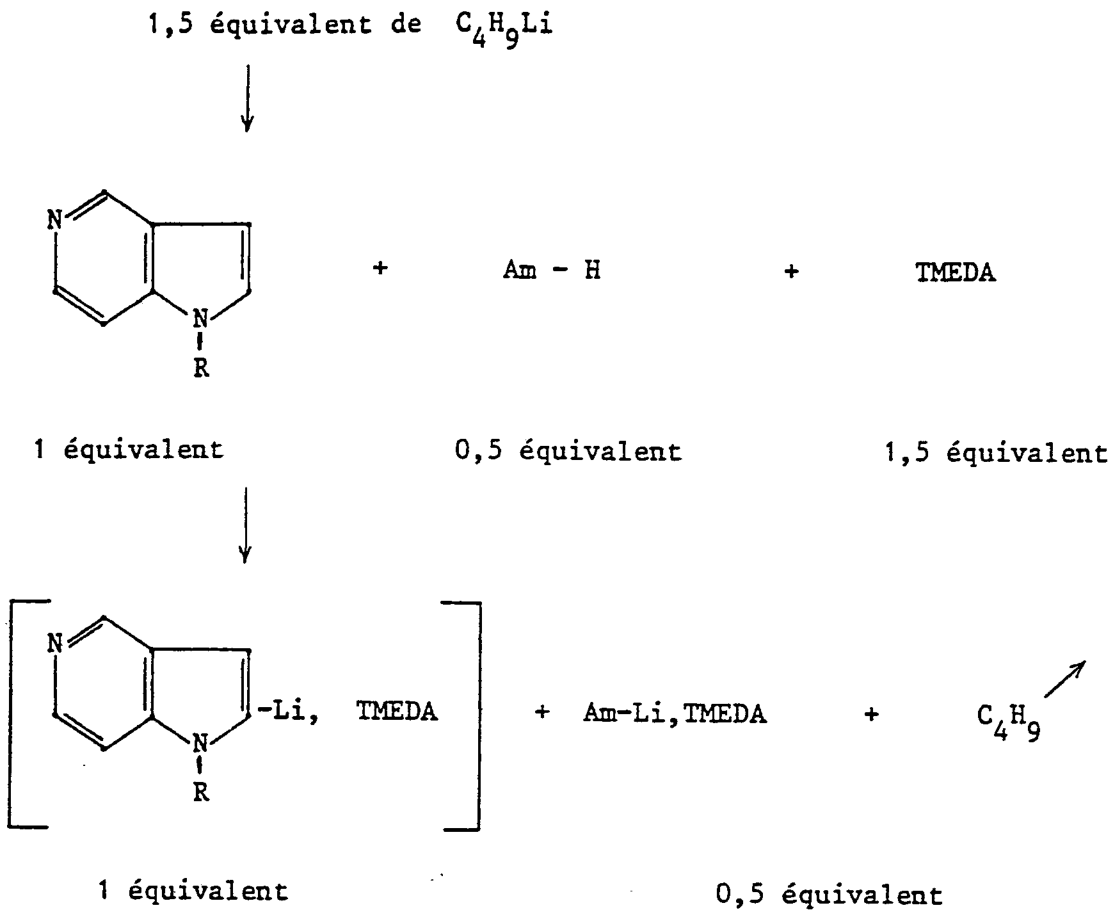


Am = diisopropylamino, tétraméthylpipéridino ...

Cette dernière méthode présente l'avantage de n'utiliser qu'un seul réacteur réfrigéré et un seul milieu réactionnel. Elle permet également de n'employer qu'une quantité minimum d'amine par rapport à la quantité de dérivé de formule I et de provoquer le déplacement de la réaction vers la formation de composé lithié par suite du dégagement de butane.

Quant à la fixation du groupement électrophile en position 2 des composés lithiens de formule II, celle-ci peut se réaliser également selon différentes procédures telles que:

addition simultanée d'amidure de lithium préparé extemporanément et de réactif capable d'engendrer un groupement électrophile à une solution du composé de formule I et de tétraméthyléthylènediamine,

addition simultanée d'amidure de lithium préparé extemporanément en présence de tétraméthyléthylènediamine et de réactif capable d'engendrer un groupement électrophile à une solution du composé de formule I,

métallation du composé de formule I telle qu'explicitée précédemment pour former le dérivé lithié de formule II puis condensation avec le réactif capable d'engendrer un groupement électrophile.

Cette dernière méthode, selon une procédure classique en deux temps est parfois préférable surtout s'il y a lieu de craindre une interaction parasite de l'agent de lithiation et du réactif capable d'engendrer un groupement électrophile.

On a pu effectuer aisément l'estimation du taux de métallation en position 2 des composés de formule I, en particulier de la benzènesulfonyl-1 1H-pyrrolo[3,2-c]pyridine en utilisant le chlorure de triméthylsilyle comme réactif capable d'engendrer un groupement électrophile, ce réactif étant inerte vis-à-vis du diisopropylamidure de lithium à −60°C.

On a utilisé à cet effet la technique suivante:

On refroidit à −60°C une solution de 0,645 g (2,5 mmoles) de benzène-sulfonyl-1 1H-pyrrolo[3,2-c]pyridine, de 0,377 ml (2,5 mmoles) de tétraméthyléthylènediamine et de 0,76 ml (6 mmoles) de chlorotriméthylsilane dans 5 ml de tétrahydrofuranne. On ajoute alors 4,5 mmoles de diisopropylamidure de lithium obtenus par réaction de 4,5 mmoles de butyllithium et 4,5 mmoles de diisopropylamine dans 5 ml de tétrahydrofuranne à température inférieure à 0°C.

La chromatographie sur couche mince montre que la réaction est complète après 15 minutes de contact à −60°C. On réchauffe le mélange à température ambiante, hydrolyse avec 10 ml d'acide

chlorhydrique 1N et extrait au dichlorométhane. Après sèchage sur sulfate de sodium et évaporation du solvant, on obtient 0,870 g d'une gomme brune.

On isole alors, par chromatographie sur silice, 0,654 g de benzènesulfonyl-1 triméthylsilyl-2 1H-pyrrolo[3,2-c]pyridine sous forme d'un solide beige, ce qui équivaut à un rendement de 82,5% (8% de produit de départ sont récupérés).

Cette méthode dans laquelle le réactif capable d'engendrer un groupement électrophile est introduit avec l'agent métallant permet une bonne estimation du taux de métallation. Le dérivé lithio-2 est piégé dès sa formation et les réactions de dégradation de ce lithien ou les réactions parasites éventuelles sont ainsi minimisées.

On a également pu estimer le taux de métallation par réaction du dérivé lithié de formule II avec $D_2O$ ou $CH_3OD$ (deutériation) selon le schéma:

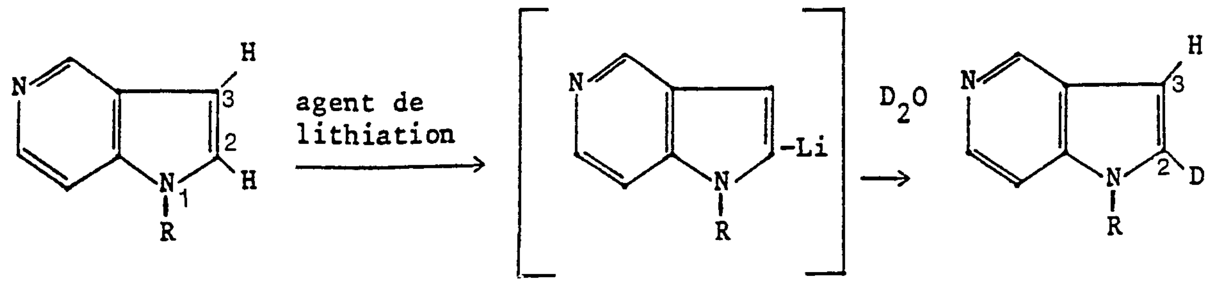


(A') (B') (C')

L'examen du spectre de résonnance magnétique nucléaire (R.M.N.) de $^1H$ des composés obtenus à l'état brut montre la disparition des deux doublets attribuables aux protons $H_2$ et $H_3$ du composé (A') au profit d'un seul singulet pour $H_3$ dans le produit (C') deutérié en position 2.

L'intégration des signaux adéquats permet d'estimer avec une bonne précision les quantités relatives des deux espèces et donc le taux de métallation correspondant à

$$\frac{\text{composé (C')}}{\text{composé (A')} + \text{composés (C')}}.$$

On a utilisé à cet effet les techniques suivantes:

a) A une solution de benzènesulfonyl-1 lithio-2 1H-pyrrolo[3,2-c]pyridine préparée comme décrit à l'Exemple 2 ci-après à partir de 5 mmoles de benzènesulfonyl-1 1H-pyrrolo[3,2-c]pyridine, 7,5 mmoles de tétraméthyléthylènediamine et 7,5 mmoles de tétraméthylpipéridide de lithium et refroidie à −70°C, on ajoute un excès d'eau lourde ($D_2O$). Après neutralisation à pH=7—8 avec de l'acide chlorhydrique 2N, on évapore le solvant sous pression réduite. On reprend le résidu dans du dichlorométhane et on lavec avec de l'eau. L'évaporation du solvant fournit 1,23 g de benzènesulfonyl-1 deutério-2 1H-pyrrolo[3,2-c]pyridine sous forme d'un solide beige-marron.

*Rendement:* 95%

*Taux de deutériation:* 85% déterminé par R.M.N.

*Spectre R.M.N.* $^1H$

6,70 ppm (s, 1H) 85%; 6,75 ppm (d, 1H), 15%; 7,1—8,1 ppm (m, 6H) 100%); 7,60 ppm (d, 1H) 15%; 8,5 ppm (d, 1H) 100%; 8,9 ppm (s, 1H) 100%.

b) A la solution de tertiobutoxycarbonyl-1 lithio-2 1H-pyrrolo[3,2-c]pyridine obtenue à l'Exemple 12 ci-après, refroidie à −70°C, on ajoute, en une fois 1,5 ml d'eau lourde. La température remonte jusqu'à −35°C. On ramène le mélange à pH=7 par addition d'acide chlorhydrique 2N puis on évapore le solvant sous vide. On reprend le résidu dans du dichlorométhane puis on lave à l'eau. On obtient ainsi 0,336 g de tertiobutoxycarbonyl-1 deutério-2 1H-pyrrolo[3,2-c]pyridine sous forme d'une huile brune.

*Rendement:* 61%

*Taux de deutériation:* 85% déterminé par R.M.N.

*Spectre R.M.N.* $^1H$

1,65 ppm (s, 9H); 6,6 ppm (s, 1H); 7,95 ppm (d, 1H); 7,45 ppm (d, 1H); 8,75 ppm (s, 1H).

Il reste à 7,6 ppm un doublet (15%) correspondant au produit de départ non deutérié en position 2.

c) On opère de la même manière avec le tertiobutoxycarbonyl-1 lithio-2 1H-pyrrolo[3,2-c]pyridine préparé selon la méthode décrite à l'Exemple 11 pour obtenir 0,713 g de tertiobutoxycarbonyl-1 deutério-2 1H-pyrrolo [3,2-c]pyridine sous forme d'une huile brune.

*Rendement:* 65%

*Taux de deutériation:* 90% déterminé par R.M.N.

On a étudié l'influence de divers facteurs sur les rendements en composé substitué en position 2 obtenus lors de la mise en oeuvre du procédé de l'invention.

A cet effet, on a réalisé la préparation de la benzènesulfonyl-1 acétyl-2 1H-pyrrolo[3,2-c]pyridine dans le tétrahydrofuranne, à température inférieure à −60°C et en faisant varier les quantités de tétraméthyléthylènediamine (TMEDA) et/ou l'agent de lithiation, le réactif capable d'engendrer un groupement électrophile étant l'anhydride acétique:

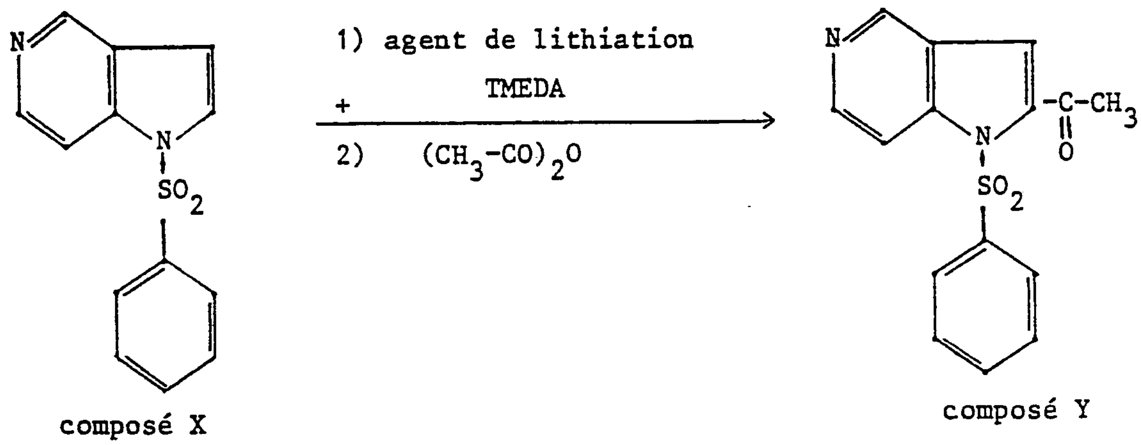


composé X

composé Y

a) *Influence de la TMEDA*

On a obtenu les résultats suivants en utilisant le diisopropylamidure de lithium (DALi) comme agent de lithiation:

| Equivalents | | | | Rendements (%) | |
|---|---|---|---|---|---|
| Composé X | DALi | TMEDA | $(CH_3CO)_2O$ | Composé X | Composé Y |
| 1 | 1,8 | 6 | 2,4 | 27 | 70 |
| 1 | 1,8 | 1 | 2,4 | 27 | 69 |
| 1 | 1,8 | 0 | 2,4 | 36 | 46 |

Ces résultats montrent la nette diminution de rendement en l'absence de TMEDA.

b) *Influence de l'agent de lithiation*

Tétraméthylpipéridide de lithium (TMPLi)

| Equivalents | | | | Rendements (%) | |
|---|---|---|---|---|---|
| Composé X | TMPLi | TMEDA | $(CH_3CO)_2O$ | Composé X | Composé Y |
| 1 | 1,2 | 6 | 2,4 | 20 | 51 |
| 1 | 1,5 | 1,5 | 2,4 | 37 | 55 |
| 1 | 1,8 | 6 | 2,4 | 28 | 65 |
| 1 | 1,2 | 0 | 2,4 | 46 | 17 |

Hexaméthyldisilylamidure de lithium (HMDALi)

| Equivalents | | | | Rendements (%) | |
|---|---|---|---|---|---|
| Composé X | HMDALi | TMEDA | $(CH_3CO)_2O$ | Composé X | Composé Y |
| 1 | 1,8 | 6 | 2,4 | 84 | 8 |

Tertiobutyllithium (t BULi)

| Equivalents | | | | Rendements (%) | |
|---|---|---|---|---|---|
| Composé X | t BULi | TMEDA | $(CH_3CO)_2O$ | Composé X | Composé Y |
| 1 | 1,2 | 1,2 | 2,4 | 58,5 | 36,5 |
| 1 | 2 | 2 | 2,4 | 31 | 49 |
| 1 | 2 | 0 | 2,4 | 35 | 41 |

Ces résultats montrent la supériorité du diisopropylamidure de lithium comme agent de lithiation et confirment l'influence favorable de la tétraméthyléthylènediamine.

Les dérivés de pyrrolo-pyridine de l'invention se révèlent particulièrement utiles comme intermédiaires de synthèse par exemple pour la préparation de composés biologiquement actifs tels que les dérivés anthelmintiques décrits dans J. Med. Chem., 1972, Vol. 15, No. 11 pp. 1168—1171 ou analogues ou encore pour la préparation de dérivés de 1H-pyrrolo[3,2-c]pyridine, mentionnés dans le brevet français No. 1.420.756.

En raison de leur structure chimique, les composés de formules I et II présentent de très intéressantes possibilités de transformation d'une part en raison de la labilité du radical R et d'autre part étant donnée les larges possibilités de substitution ou de modification offertes au niveau de la position 2.

En outre, et c'est là un de leurs avantages les plus importants, les dérivés de pyrrolo-pyridine de l'invention sont obtenus, comme décrit précédemment, selon des méthodes faciles à mettre en oeuvre et applicables industriellement.

Les Exemples non limitatifs suivants illustrent l'invention:

## Exemple 1

Préparation de la benzènesulfonyl-1 1H-pyrrolo[3,2-c]pyridine

On place dans 850 ml de dichlorométhane, 33,12 g (0,28 mole) de 1H-pyrrolo[3,2-c]pyridine, 28 g (0,70 mole) d'hydroxyde de sodium pulvérisé et 1,09 g (0,0032 mole) de sulfate acide de tétrabutylammonium $[(n\text{-}C_4H_9)_4N,HSO_4]$ comme catalyseur de transfert de phase puis on agite la solution vigoureusement.

On ajoute ensuite, en une heure, 53,7 ml (0,42 mole) de chlorure de benzènesulfonyle et l'on enregistre une élévation de température de 20° à 40°C. On maintient l'agitation pendant une heure après la fin de l'addition.

On essore alors l'excès d'hydroxyde de sodium et le chlorure de sodium formé et on lave le filtrat à l'eau jusqu'à pH=7—8.

Après sèchage sur sulfate de magnésium et décoloration partielle sur charbon actif, on élimine le solvant sous vide réduit.

On obtient ainsi 68,4 g de benzènesulfonyl-1 1H-pyrrolo[3,2-c]pyridine, sous forme d'un solide brun, dont la pureté est de l'ordre de 90%.

*Rendement:* 83—85% après purification par filtration sur silice.

*P.F.*: 127°C

*Spectre I.R.*

ν CH= 3140—3120 $cm^{-1}$

ν C=O 1600 cm || 1

ν $SO_2$ 1370 $cm^{-1}$

*Spectre R.M.N.* $^1H$

6,8 ppm (d, 1H); 7,5—8 ppm (m, 5H); 7,6 ppm (d, 1H); 8,5 ppm (d, 1H); 8,9 ppm (s, 1H)
*Analyse*

| | C% | H% | N% | S% |
|---|---|---|---|---|
| Calculé | 60,45 | 3,90 | 10,85 | 12,41 |
| Trouvé | 60,16 | 3,81 | 10,47 | 12,16 |

Exemple 2

Preparation de la benzènesulfonyl-1 lithio-2 1H-pyrrolo[3,2-c]pyridine

On refroidit à −60°C', une solution de 1,3 g (5 mmoles) de benzènesulfonyl-1 1H-pyrrolo[3,2-c]pyridine et de 0,75 ml (5 mmoles) de tétraméthyléthylènediamine dans 10 ml de tétrahydrofuranne. On ajoute alors en quelques minutes 9 mmoles de diisopropylamidure de lithium (préparé par réaction de 9 mmoles de butyl lithium avec 9 mmoles de diisopropylamine dans 5 ml de tétrahydrofuranne à température inférieure à 0°C) de façon que la température du milieu n'excède pas −40°C.

On agite la solution à −60°C pendant 30 minutes.

On obtient ainsi une solution de benzènesulfonyl-1 lithio-2 1H-pyrrolo [3,2-c]pyridine que l'on utilise telle quelle.

Exemple 3

Préparation de la benzènesulfonyl-1 acétyl-2 1H-pyrrolo[3,2-c]pyridine

A la solution de benzènesulfonyl-1 lithio-2 1H-pyrrolo[3,2-c]pyridine obtenue à l'Exemple 2, refroidie vers −70°C, on ajoute en une fois 1,13 ml (12 mmoles; 2,4 équivalents) d'anhydride acétique et on réchauffe le mélange à température ambiante.

On hydrolyse alors le milieu avec 50 ml d'acide chlorhydrique 1N puis on l'extrait au dichlorométhane, ce qui fournit 1,7 g de produit brut.

Après chromatographie sur silice, on isole 0,35 g (environ 27%) de produit de départ et 1,03 g de benzènesulfonyl-1 acétyl-2 1H-pyrrolo[3,2-c]pyridine.

*Rendement:* 69%
*P.F.:* 205°C
*Spectre I.R.* (KBr)
ν O= 1685 $cm^{-1}$
ν $SO_2$ 1375 $cm^{-1}$

*Spectre R.M.N.*
2,9 ppm (s, 3H); 7,5—9 ppm (m, 8H); 9,4 ppm (s, 1H).
*Analyse*

**Analyse**

| | C% | H% | N% | S% |
|---|---|---|---|---|
| Calculé | 59,99 | 4,03 | 9,33 | 10,68 |
| Trouvé | 59,61 | 4,02 | 9,33 | 10,95 |

De la même manière que celle décrite ci-dessus, on a préparé la benzène-sulfonyl-1 triméthylsilyl-2 1H-pyrrolo[3,2-c]pyridine à partir de chlorure de triméthylsilyle et de benzènesulfonyl-1 lithio-2 1H-pyrrolo[3,2-c]pyridine.

*P.F.:* 125°C
*Spectre R.M.N.*
0,5 ppm (s, 9H); 7 ppm (s, 1H); 7,3—7,9 ppm (m, 6H); 8,4 ppm (d, 1H); 8,9 ppm (s, 1H)
*Analyse*

| | C% | H% | N% | S% |
|---|---|---|---|---|
| Calculé | 58,18 | 5,45 | 8,48 | 9,70 |
| Trouvé | 57,98 | 5,48 | 8,54 | 9,52 |

Exemple 4

Préparation de la benzènesulfonyl-1 lithio-2 1H-pyrrolo[3,2-c]pyridine

On met en solution dans 15 ml de tétrahydrofuranne 1,3 g (5 mmoles) de benzènesulfonyl-1 1H-pyrrolo[3,2-c]pyridine et 4,5 ml (30 mmoles) de tétraméthyléthylènediamine. A cette solution refroidie à −70°C, on ajoute, en 5 à 10 minutes, 6 mmoles de tétraméthylpipéridide de lithium dans 5 ml de tétrahydrofuranne (préparé par réaction de 6 mmoles de butyllithium avec 6 mmoles de tétraméthylpipéridine dans le tétrahydrofuranne à température inférieure à 0°C).

On poursuit l'agitation pendant 1 heure entre −65°C et −40°C puis on réchauffe la solution jusqu'à −20°C.

On obtient ainsi une solution de benzènesulfonyl-1 lithio-2 1H-pyrrolo[3,2-c]pyridine que l'on utilise brute.

Exemple 5

Préparation de la benzènesulfonyl-1 éthoxycarbonyl-2 1H-pyrrolo[3,2-c] pyridine

A la solution de benzènesulfonyl-1 lithio-2 1H-pyrrolo[3,2-c]pyridine obtenue à l'Exemple 4, refroidie à −70°C, on ajoute 1,15 ml (12 mmoles) de chloroformiate d'éthyle et on réchauffe ensuite à température ambiante.

On neutralise le milieu réactionnel avec une solution d'acide chlorhydrique 1N et on extrait au dichlorométhane. Après sèchage de la phase organique sur sulfate de sodium et évaporation du solvant, on obtient 1,85 g de produit brut.

Une chromatographie sur silice fournit alors 1,23 de benzènesulfonyl-1 éthoxycarbonyl-2 1H-pyrrolo[3,2-c]pyridine sous forme d'un solide beige.

*Rendement:* 74,5%

*P.F.*: 145°C

*Spectre I.R.*

ν C=O 1730 $cm^{-1}$

ν $SO_2$ 1370 $cm^{-1}$

*Spectre R.M.N.*

1,4 ppm (t, 3H); 4,4 ppm (q, 2H); 7,3 ppm (s, 1H); 7,4—8,3 ppm (m, 6H); 8,6 ppm (d, 1H); 9 ppm (s, 1H).

*Analyse*

| | C% | H% | N% | S% |
|---|---|---|---|---|
| Calculé | 58,17 | 4,27 | 8,48 | 9,71 |
| Trouvé | 57,91 | 4,25 | 8,21 | 9,56 |

De la même manière que celle décrite ci-dessus, on a préparé la benzènesulfonyl-1 méthoxycarbonyl-2 1H-pyrrolo[3,2-c]pyridine à partir de chloroformiate de méthyle et de benzènesulfonyl-1 lithio-2 1H-pyrrolo[3,2-c]pyridine.

*P.F.*: 166°C

*Spectre I.R.* (KBr)

ν C=O 1720 $cm^{-1}$

ν $SO_2$ 1377 $cm^{-1}$

*Spectre R.M.N.* ($CDCl_3$/tétraméthylsilane/acide trifluoroacétique) 3,9 ppm (s, 3H); 7,3—9,4 ppm (m, 9H).

Exemple 6

Préparation de la benzènesulfonyl-1 lithio-2 1H-pyrrolo[3,2-c]pyridine

On met en solution, dans 15 ml de tétrahydrofuranne, 1,3 g (5 mmoles) de benzènesulfonyl-1 1H-pyrrolo[3,2-c]pyridine et 9 mmoles de tétraméthyléthylènediamine. A cette solution refroidie à température inférieure à −60°C, on ajoute, en 5 à 10 minutes, 9 mmoles de diisopropylamidure de lithium, préparé par réaction de 9 mmoles de butyllithium avec 9 mmoles de diisopropylamine dans le tétrahydrofuranne.

On poursuit l'agitation pendant 15 à 30 minutes à −60°C et on contrôle par chromatographie sur couche mince que la métallation est complète par réaction d'une partie aliquote du milieu réactionnel avec un excès de triméthylsilane.

On obtient ainsi une solution de benzènesulfonyl-1 lithio-2 1H-pyrrolo [3,2-c]pyridine que l'on utilise brute.

Exemple 7

Préparation de dérivés de benzènesulfonyl-1 1H-pyrrolo[3,2-c]pyridine substitués en position 2 par un groupement électrophile

A la solution de benzènesulfonyl-1 lithio-2 1H-pyrrolo[3,2-c]pyridine obtenue à l'Exemple 6, on ajoute 12 à 15 mmoles de réactif capable d'engendrer un groupement électrophile et on suit l'avancement de la réaction par chromatographie sur couche mince.

On neutralise le milieu réactionnel par l'acide chlorhydrique 1N et on extrait au dichlorométhane.

Après sèchage et évaporation de la phase organique, on purifie le résidu par filtration sur silice.

De cette manière on a obtenu les composés suivants:

a) Benzènesulfonyl-1 formyl-2 1H-pyrrolo[3,2-c]pyridine.

*Réactif produisant le groupement électrophile:* formiate d'éthyle.
*Rendement:* 52%
*P.F.*: 164°C
*Spectre I.R.* (KBr)
ν C=O 1686 $cm^{-1}$
ν C=H 2930 $cm^{-1}$
ν $SO_2$ 1378 $cm^{-1}$
*Spectre R.M.N.* ($CDCl_3$/TMS)
7,2—9,1 ppm (m,9H); 10,45 ppm (s, 1H).

b) Benzènesulfonyl-1 hydroxycarbonyl-2 1H-pyrrolo[3,2-c]pyridine
*Réactif produisant le groupement électrophile:* anhydride carbonique solide
*Rendement:* 80%
*Spectre I.R.*
ν C=O 1600 $cm^{-1}$
ν $SO_2$ 1384 $cm^{-1}$

c) Benzènesulfonyl-1 (hydroxy-1 éthyl)-2 1H-pyrrolo[3,2-c]pyridine.
*Réactif produisant le groupement électrophile:* acétaldéhyde
*Rendement:* 48%
*P.F.*: 185°C
*Spectre R.M.N.:* ($CDCl_3$/$DMSOD_6$/εTFA/TMS)
1,55 ppm (d, J=6,5 Hz, 3H); 3,45 ppm (q, J=6,5 Hz, 1H); 7,2 ppm (s, 1H); 7,5—9,4 ppm (m, 8H).

d) Benzènesulfonyl-1 (hydroxy-1 phényl-1 éthyl)-2 1H-pyrrolo[3,2-c]pyridine
*Réactif produisant le groupement électrophile:* acétophénone
*Rendement:* 69%
*P.F.*: 150°C
*Spectre R.M.N.* ($CDCl_3$/TMS)
1,95 ppm (s, 3H); 7 ppm (s, 1H); 7,1—7,6 ppm (m, 5H); 7,95 ppm (d, J=6 Hz, 1H); 8,5 ppm (d, J=6 Hz, 1H); 8,9 ppm (s, 1H).

e) Benzènesulfonyl-1 diéthylaminocarbonyl-2 1H-pyrrolo[3,2-c]pyridine
*Réactif produisant le groupement électrophile:* chlorure de diéthylcarbamoyle
*Rendement:* 20%
*I.R.* (film)
ν C=O 1730 $cm^{-1}$
ν $SO_2$ 1377 $cm^{-1}$
*R.M.N.* ($CDCl_3$/TMS)
1,25 ppm (m, 6H); 3,4 ppm (m, 4H); 7,2—9 ppm (m, 9H).

f) Benzènesulfonyl-1 éthyl-2 1H-pyrrolo[3,2-c]pyridine
*Réactif produisant le groupement électrophile:* iodure d'éthyle
*Rendement:* 21%
*P.F.*: 121°C
*R.M.N.* ($CDCl_3$/TMS)
1,4 ppm (t, J=7 Hz, 3H); 3 ppm (q, J=7 Hz, 2H); 6,45 ppm (s, 1H); 7,2—7,9 ppm (m, 5H); 8,05 ppm (d, J=6 Hz, 1H); 8,4 ppm (d, J=6 Hz, 1H); 8,75 ppm (s, 1H)

## Exemple 8

Préparation du benzènesulfonyl-1 lithio-2 1H-pyrrolo[3,2-c]pyridine

On refroidit à −70°C une solution de 0,645 g (2,5 mmoles) de benzènesulfonyl-1 1H-pyrrolo[3,2-c]-pyridine et de 0,755 ml (5 mmoles) de tétraméthyléthylènediamine dans 5 ml de tétrahydrofuranne et on y ajoute 5 mmoles de tertiobutyl lithium dans le pentane de façon que la température n'excède pas −60°C.

On agite alors le milieu réactionnel de manière à obtenir une solution de benzènesulfonyl-1 lithio-2 1H-pyrrolo[3,2-c]pyridine que l'on utilise telle quelle.

## Exemple 9

Préparation de la benzènesulfonyl-1 acétyl-2 1H-pyrrolo[3,2-c]pyridine

On porte à −70°C la solution de benzènesulfonyl-1 lithio-2 1H-pyrrolo [3,2-c]pyridine obtenue à l'Exemple 8 et on y ajoute en une seule fois 0,5 g (6 mmoles) d'anhydride acétique.

Après réchauffement à température ambiante et isolement comme dans l'Exemple 3, la

chromatographie fournit 0,365 g de benzènesulfonyl-1 acétyl-2 1H-pyrrolo[3,2-c]pyridine.
*Rendement:* 49%

Exemple 10

Préparation du tertiobutoxycarbonyl-1 1H-pyrrolo[3,2-c]pyridine

En suivant le même procédé que décrit dans l'Exemple 1 mais au départ de 22,6 g (0,2 mole) de 1H-pyrrolo[3,2-c]pyridine et de 55 ml (0,24 mole) de dicarbonate de tertiobutyle, on obtient 42,5 g d'une huile brune qui cristallise au réfrigérateur.

Par purification chromatographie d'un échantillon analytique, on obtient la tertiobutoxycarbonyl-1 1H-pyrrolo[3,2-c]pyridine sous forme d'une poudre blanc-crème.

*P.F.*: 65°C
*Spectre I.R.*
ν C=O 1735 $cm^{-1}$
*Spectre R.M.N.*
1,65 ppm (s, 9H); 6,6 ppm (d, 1H); 7,65 ppm (d, 1H); 7,95 ppm (d, 1H); 8,45 ppm (d, 1H); 8,75 ppm (s, 1H).

| | C% | H% | N% |
|---|---|---|---|
| Calculé | 66,04 | 6,47 | 12,83 |
| Trouvé | 66,20 | 6,51 | 13,10 |

Exemple 11

Préparation du tertiobutoxycarbonyl-1 lithio-2 1H-pyrrolo[3,2-c]pyridine

On refroidit à −70°C une solution de 1,092 g (5 mmoles) de tertiobutoxycarbonyl-1 1H-pyrrolo[3,2-c]pyridine, 1,14 ml (7,5 mmoles) de tétraméthyléthylènediamine et 0,42 ml (2,5 mmoles) de tétraméthylpipéridine dans 7,6 ml de tétrahydrofuranne.

On ajoute alors, en 15 minutes, 7,5 mmoles de butyllithium en maintenant la température en dessous de −60°C et on agite, pendant 30 minutes à cette température la solution rouge-brun obtenue.

On obtient ainsi une solution de tertiobutoxycarbonyl-1 lithio-2 1H-pyrrolo[3,2-c]pyridine que l'on utilise telle quelle.

Exemple 12

Préparation du tertiobutoxycarbonyl-1 lithio-2 1H-pyrrolo[3,2-c]pyridine

On refroidit à −70°C une solution de 0,549 g (2,5 mmoles de tertiobutoxycarbonyl-1 1H-pyrrolo[3,2-c]pyridine et de 0,76 ml (5 mmoles) de tétraméthyléthylènediamine dans 2,5 ml de tétrahydrofuranne. On ajoute ensuite en quelques minutes 5 mmoles de tétraméthylpipéridide de lithium (préparé par réaction de 5 mmoles de butyllithium avec 5 mmoles de tétraméthylpipéridine dans 2,5 ml de tétrahydrofuranne à température inférieure à −20°C) de façon que la température du milieu n'excède pas −60°C.

On agite la solution rouge-brun ainsi obtenue pendant 30 minutes à température inférieure ou égale à −60°C.

On obtient ainsi une solution de tertiobutoxycarbonyl-1 lithio-2 1H-pyrrolo[3,2-c]pyridine que l'on utilise telle quelle.

Exemple 13

Preparation du tertiobutoxycarbonyl-1 triméthylsilyl-2 1H-pyrrolo[3,2-c]pyridine

A la solution de tertiobutoxycarbonyl-1 lithio-2 1H-pyrrolo[3,2-c]pyridine, obtenue à l'Exemple 12, refroidie à −70°C, on ajoute 0,48 ml (3,8 mmoles) de chlorure de triméthylsilyle. La température remonte jusqu'à −50°C. On rechauffe le mélange à température ambiante et on amène le pH à 7 par addition d'acide chlorhydrique 2N. On évapore le solvant sous pression réduite et on reprend le résidu dans le dichlorométhane. On lave à l'eau et on sèche sur sulfate de magnésium. On évapore le solvant, ce qui fournit 0,65 g d'un solide blanc cassé.

Après purification chromatographie sur silice, on obtient 0,561 g de tertiobutoxycarbonyl-1 triméthylsilyl-2 1H-pyrrolo[3,2-c]pyridine sous forme d'un solide blanc.

*Rendement:* 78%
*P.F.*: 112°C
*Spectre I.R.*
ν C=O 1735 $cm^{-1}$
*Spectre R.M.N.*
0,4 ppm (s, 9H); 1,65 ppm (s, 9H); 6,85 ppm (s, 1H); 7,95 ppm (d, 1H); 8,45 ppm (d, 1H); 8,7 ppm (s, 1H).

De la même manière que précédemment au départ de tertiobutoxycarbonyl-1 lithio-2 1H-pyrrolo[3,2-c]pyridine et de benzaldéhyde, on obtient le tertiobutoxycarbonyl-1 (hydroxy-1 phényl-1 méthyl)-2 1H-pyrrolo[3,2-c]pyridine.

*Rendement:* 76%
*Spectre I.R.*
ν C=O 1735 $cm^{-1}$
*Spectre R.M.N.*
1,4 ppm (s, 9H); 6,60 ppm (s, 1H); 7,1—7,9 ppm (m, 7H); 8,45 ppm (d, 1H); 9,1 ppm (s, 1H)

**Revendications**

1. Procédé pour la fixation d'un groupement électrophile en position 2 de dérivés de 1H-pyrrolo[3,2-c]pyridine caractérisé en ce que:

a) l'on fait réagir à température ambiante la 1H-pyrrolo[3,2-c]pyridine dans un solvant, au moyen d'un hydroxyde de métal alcalin et en présence d'un catalyseur de transfert de phase puis, à une température comprise entre la température ambiante et 40°C, avec un halogénure de formule générale:

R—Hal

dans laquelle R représente un groupement méthoxy méthyle, benzyloxyméthyle, benzènesulfonyle, p-toluènesulfonyle ou tertiobutoxycarbonyle et Hal représente un atome de chlore, de brome ou d'iode, pour obtenir les dérivés N-protégés de 1H-pyrrolo[3,2-c]pyridine de formule générale:

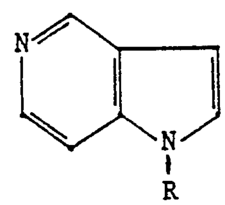

I

dans laquelle R a la même signification que précédemment,

b) l'on fait réagir dans un solvant le dérivé N-protégé de 1H-pyrrolo [3,2-c]pyridine obtenu au paragraphe a) avec un agent de lithiation lequel est soit un amidure de lithium, soit un alkyl lithium, à une température comprise entre −80°C et −20°C et en présence de tétraméthyléthylénediamine, pour obtenir les dérivés lithio-2 de formule générale:

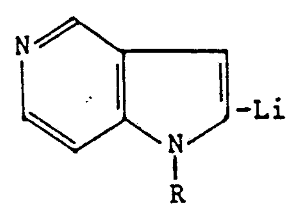

II

dans laquelle R a la même signification que précédemment,

c) l'on condense dans un solvant et à une température comprise entre −80°C et la température ambiante, le dérivé lithio-2 obtenu au paragraphe b) avec un réactif capable d'engendrer un groupement électrophile, pour obtenir des dérivés de 1H-pyrrolo[3,2-c]pyridine de formule I substitués en position 2 par un groupement électrophile.

2. Procédé pour la préparation de dérivés de 1H-pyrrolo[3,2-c]pyridine de formule générale:

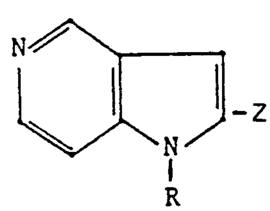

III

dans laquelle:

R représente un groupement methoxyméthyle, benzyloxyméthyle, benzènesulfonyle, p-toluènesulfonyle ou tertiobutoxycarbon,

Z représente:

un atome d'hydrogène

un atome de lithium

un groupement alkyle inférieur

un groupement de formule:

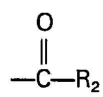

(A)

dans lequel $R_2$ représente hydrogène, un groupement alkyle inférieur, un groupement $—OR_3$ dans lequel $R_3$ représente hydrogène ou un groupement alkyle inférieur ou encore $R_2$ représente un groupement $—N(R_4)_2$ dans lequel $R_4$ représente un groupement alkyle inférieur,

un groupement de formule:

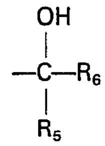 (B)

dans lequel $R_5$ représente hydrogène ou un groupement alkyle inférieur et $R_6$ représente phényle ou un groupement alkyle inférieur,

un groupement silyle de formule:

$$Si(R_6)_3 \quad (C)$$

dans laquelle $R_6$ a la même signification que précédemment, caractérisé en ce que;

a) l'on fait réagir à température ambiante la 1H-pyrrolo[3,2-c]pyridine dans un solvant au moyen d'un hydroxyde de métal alcalin et en présence d'un catalyseur de transfert de phase puis, à une température comprise entre la température ambiante et 40°C, avec un halogénure de formule générale:

$$R—Hal$$

dans laquelle R a la même signification que précédemment et Hal représente un atome de chlore, de brome ou d'iode, pour obtenir les dérivés N-protégés de 1H-pyrrolo[3,2-c]pyridine de formule III dans laquelle Z représente un atome d'hydrogène,

b) l'on fait réagir dans un solvant le dérivé N-protégé de 1H-pyrrolo [3,2-c]pyridine obtenu au paragraphe a) avec un agent de lithiation lequel est soit un amidure de lithium, soit un alkyl lithium, à une température comprise entre −80°C et −20°C et en présence de tétraméthyléthylènediamine, pour obtenir les dérivés lithio-2 de formule III dans laquelle Z représente un atome de lithium.

c) l'on condense dans un solvant et à une température comprise entre −80°C et la température ambiante, le dérivé lithio-2 obtenu au paragraphe b) avec un réactif capable d'engendrer un groupement électrophile choisi parmi le groupe formé par:

un halogénure d'alkyle de formule générale:

$$R_4—Hal$$

dans laquelle $R_4$ a la même signification que précédemment et Hal représente un atome d'halogène pour obtenir les composés de formule III dans laquelle Z représente un groupement alkyle inférieur,

un ester de formule générale:

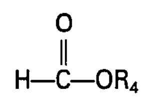

dans laquelle $R_4$ a la même signification que précédemment ou un anhydride de formule générale:

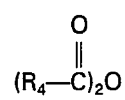

dans laquelle $R_4$ a la méme signification que précédemment ou l'anhydride carbonique, ou un halogénure de formule générale:

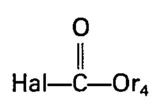

dans laquelle Hal et $R_4$ ont la même signification que précédemment ou un halogénure de formule générale:

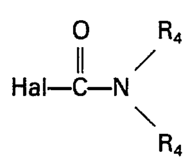

dans laquelle Hal et $R_4$ ont la même signification que précédemment, pour obtenir les composés de formule III dans laquelle Z représente un groupement de formule (A)

un aldéhyde ou une cétone de formule générale:

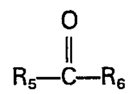

dans laquelle $R_5$ et $R_6$ ont la même signification que précédemment, pour obtenir les composés de formule III dans laquelle Z représente un groupement de formule (B)

un halogénure de silyle de formule générale:

$$Hal—Si(R_6)_3$$

dans laquelle Hal et $R_6$ ont la même signification que précédemment pour obtenir les composés de formule III dans laquelle Z représente un groupement (C).

3. Procédé selon la revendication 1 ou 2 caractérisé en ce que R (le groupement protecteur labile) est un groupement méthoxyméthyle, benzyloxyméthyle, benzènesulfonyle ou p-toluènesulfonyle ou tertiobutoxycarbonyle (30C).

4. Procédé selon la revendication 3 caractérisé en ce que le groupement protecteur labile est un groupement benzènesulfonyle ou tertiobutoxycarbonyle.

5. Procédé selon la Revendication 1 ou 2 caractérisé en ce que l'amidure de lithium est le diisopropylamidure de lithium.

6. Procédé selon la Revendication 1 ou 2 caractérisé en ce que l'on utilise de 1 à 6 équivalents de tétraméthyléthylènediamine par équivalent de 1H-pyrrolo[3,2-c]pyridine N-protégée.

7. Procédé selon la Revendication 1 ou 2 caractérisé en ce que le solvant est un éther ou un mélange éther/hydrocarbure.

8. Procédé selon la Revendication 7 caractérisé en ce que l'éther est le tétrahydrofuranne et le mélange éther/hydrocarbure est un mélange tétrahydrofuranne/pentane.

9. Dérivés de 1H-pyrrolo[c]pyridine de formule générale:

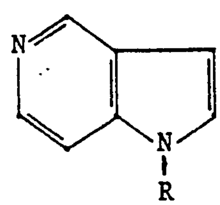

dans laquelle R représente un groupement méthoxyméthyl, benzyloxyméthyle, benzènesulfonyle, p-toluènesulfonyle ou tertiobutoxycarbonyle.

10. Benzènesulfonyl-1 1H-pyrrolo[3,2-c]pyridine.

11. Tertiobutoxycarbonyl-1 1H-pyrrolo[3,2-c]pyridine.

**Patentansprüche**

1. Verfahren zur Bindung einer elektrophilen Gruppe in 2-Stellung von Derivaten des 1H-Pyrrolo[3,2-c]pyridins, gekennzeichnet durch

a) Umsetzung von 1H-Pyrrolo[3,2-c]pyridin in einem Lösungsmittel mittels eines Alkalimetallhydroxids sowie in Gegenwart eines Phasenübertragungskatalysators bei Raumtemperatur sowie anschließend bei einer Temperatur zwischen Raumtemperatur und 40°C mit einem Halogenid der allgemeinen Formel

$$R—Hal,$$

in der R Methoxymethyl, Benzyloxymethyl, Benzolsulfonyl, p-Toluolsulfonyl oder t-Butoxycarbonyl und Hal ein Chloratom, Bromatom oder Jodatom bedeuten, zu den N-geschützten Derivaten des 1H-Pyrrolo[3,2-c]pyridins der allgemeinen Formel

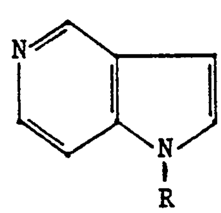

I

mit R wie oben definiert,

b) Umsetzumg des in Stufe a) erhaltenen N-geschützten Derivats des 1H-Pyrrolo[3,2-c]pyridins in einem Lösungsmittel mit einem Lithiumamid oder einem Alkyllithium als Mittel zur Einführung von Lithium bei einer Temperatur zwischen −80 und −20°C sowie in Gegenwart von Tetramethylethylendiamin zu den 2-Lithium-Derivaten der allgemeinen Formel

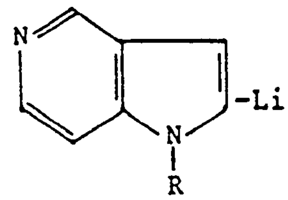

II

mit R wie oben
und

c) Kondensation des in Stufe b) erhaltenen 2-Lithium-Derivats in einem Lösungsmittel sowie bei einer Temperatur zwischen −80°C und der Raumtemperatur mit einem zur Einführung einer elektrophilen Gruppe geeigneten Reagens zu Derivaten des 1H-Pyrrolo[3,2-c]pyridins der Formel I, die in 2-Stellung mit einer elektrophilen Gruppe substituiert sind.

2. Verfahren zur Herstellung von Derivaten des 1H-Pyrrolo[3,2-c]pyridins der allgemeinen Formel

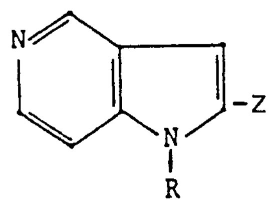

III

in der bedeuten:

R Methoxymethyl, Benzyloxymethyl, Benzolsulfonyl, p-Toluolsulfonyl oder t-Butoxycarbonyl und

Z ein Wasserstoffatom, ein Lithiumatom, eine niedere Alkylgruppe, eine Gruppe der Formel

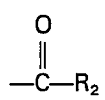

(A),

in der $R_2$ Wasserstoff, eine niedere Alkylgruppe, eine Gruppe $-OR_3$, in der $R_3$ Wasserstoff oder eine niedere Alkylgruppe darstellt, oder eine Gruppe $-N(R_4)_2$, in der $R_4$ eine niedere Alkylgruppe darstellt, bedeutet,

eine Gruppe der Formel

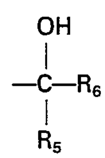

(B),

in der $R_5$ Wasserstoff oder eine niedere Alkylgruppe und

$R_6$ Phenyl oder eine niedere Alkylgruppe bedeuten,

oder eine Silylgruppe der Formel

$$-Si(R_6)_3 \quad (C),$$

in der $R_6$ die obige Bedeutung besitzt, gekennzeichnet durch

a) Umsetzung von 1H-Pyrrolo[3,2-c]pyridin in einem Lösungsmittel mittels eines Alkalimetallhydroxids sowie in Gegenwart eines Phasentransferkatalysators bei Raumtemperatur sowie anschließend bei einer Temperatur zwischen Raumtemperatur und 40°C mit einem Halogenid der allgemeinen Formel

$$R-Hal,$$

in der R die oben angegebene Bedeutung besitzt und Hal ein Chloratom, Bromatom oder Jodatom darstellt, zu den N-geschützten Derivaten des 1H-Pyrrolo[3,2-c]pyridins der Formel III, in der Z ein Wasserstoffatom darstellt,

b) Umsetzung des in Stufe a) erhaltenen N-geschützten Derivats des 1H-Pyrrolo[3,2-c]pyridins in einem Lösungsmittel mit einem Lithiumamid oder einem Alkyllithium als Mittel zur Einführung von Lithium bei einer Temperatur zwischen −80 und −20°C sowie in Gegenwart von Tetramethylendiamin zu den 2-Lithium-Derivaten der Formel III, in der Z ein Lithiumatom bedeutet,

c) Kondensation des in Stufe b) erhaltenen 2-Lithium-Derivats in einem Lösungsmittel bei einer

Temperatur zwischen −80°C und Raumtemperatur mit einem zur Einführung einer elektrophilen Gruppe geeigneten Reagens das ausgewählt ist unter

Alkylhalogeniden der allgemeinen Formel

$R_4$—Hal,

in der $R_4$ dasselbe wie oben und Hal ein Halogenatom bedeuten, zu den Verbindungen der Formel III, in der Z eine niedere Alkylgruppe darstellt,

Estern der allgemeinen Formel

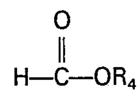

mit $R_4$ wie oben
oder einem Anhydrid der allgemeinen Formel

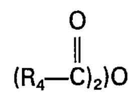

mit $R_4$ wie oben
oder Kohlendioxid oder einem Halogenid der allgemeinen Formel

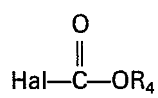

mit Hal und $R_4$ wie oben oder einem Halogenid der allgemeinen Formel

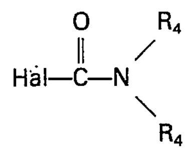

mit Hal und $R_4$ wie oben zu den Verbindungen der Formel III, in der Z eine Gruppe der Formel A bedeutet, Aldehyden oder Ketonen der allgemeinen Formel

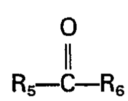

mit $R_5$ und $R_6$ wie oben zu Verbindungen der Formel III, in der Z eine Gruppe der Formel B darstellt, oder

Silylhalogeniden der allgemeinen Formel

Hal—Si$(R_6)_3$

mit Hal und $R_6$ wie oben zu den Verbindungen der Formel III, in der Z eine Gruppe C darstellt.

3. Verfahren nach Anspruch 1 oder 2, dadurch gekennzeichnet, daß die eine labile Schutzgruppe darstellende Gruppe R Methoxymethyl, Benzyloxymethyl, Benzolsulfonyl, p-Toluolsulfonyl oder t-Butoxycarbonyl (BOC) ist.

4. Verfahren nach Anspruch 3, dadurch gekennzeichnet, daß die labile Schutzgruppe Benzolsulfonyl oder t-Butoxycarbonyl ist.

5. Verfahren nach Anspruch 1 oder 2, dadurch gekennzeichnet, daß das Lithiumamid Lithiumdiisopropylamid ist.

6. Verfahren nach Anspruch 1 oder 2, dadurch gekennzeichnet, daß 1 bis 6 Äquivalente Tetramethylethylendiamin pro Äquivalent des N-geschützten 1H-Pyrrolo[3,2-c]pyridins verwendet werden.

7. Verfahren nach Anspruch 1 oder 2, dadurch gekennzeichnet, daß das Lösungsmittel ein Ether oder ein Ether/Kohlenwasserstoff-Gemisch ist.

8. Verfahren nach Anspruch 7, dadurch gekennzeichnet, daß der Ether Tetrahydrofuran und das Ether/Kohlenwasserstoff-Gemisch ein Tetrahydrofuran/Pentan-Gemisch sind.

9. Derivate des 1H-Pyrrolo[3,2-c]pyridins der allgemeinen Formel

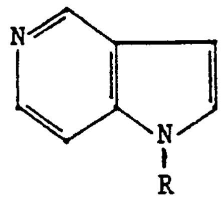

in der R Methoxymethyl, Benzyloxymethyl, Benzolsulfonyl, p-Toluolsulfonyl oder t-Butyloxycarbonyl bedeutet.

10. 1-Benzolsulfonyl-1H-pyrrolo[3,2-c]pyridin.

11. 1-t-Butoxycarbonyl-1H-pyrrolo[3,2-c]pyridin.

## Claims

1. Method of fixing an electrophilic group in the 2-position of 1H-pyrrolo[3,2-c]pyridine derivatives characterised in that:

a) 1H-pyrrolo[3,2-c]pyridine is reacted, at room temperature and in a solvent, with an alkali metal hydroxide and in the presence of an interphase transfer catalyst then, at a temperature between room temperature and 40°C, with a halide of general formula:

R—Hal

in which R represents a methoxymethyl, benzyloxymethyl, benzenesulphonyl, p-toluenesulphonyl or tert.butoxycarbonyl group and Hal represents a chlorine, bromine or iodine atom, to obtain the N-protected 1H-pyrrolo[3,2-c]pyridine derivatives of general formula:

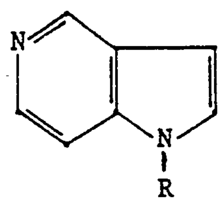

I

in which R has the same meaning as above,

b) the N-protected 1H-pyrrolo[3,2-c]pyridine obtained in paragraph a) above is reacted in a solvent, at a temperature between −80°C and −20°C and in the presence of tetramethylethylenediamine, with a lithiation agent which is either a lithium amide or an alkyl lithium, to obtain the 2-lithio derivatives of general formula:

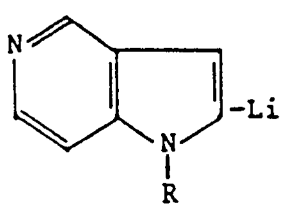

II

in which R has the same meaning as above,

c) the 2-lithio derivative obtained in paragraph b) above is condensed in a solvent and at a temperature between −80°C and room temperature with a reagent capable of producing an electrophilic group, to obtain the 1H-pyrrolo[3,2-c]pyridine derivatives of formula I substituted in the 2-position by an electrophilic group.

2. Process for preparing 1H-pyrrolo[3,2-c]pyridine derivatives of general formula:

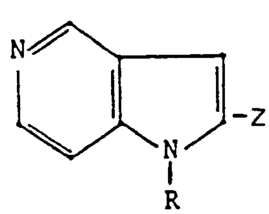

III

in which:

R represents a methoxymethyl, benzyloxymethyl, benzenesulphonyl, p-toluene-sulphonyl or tert. butoxycarbonyl group

Z represents a hydrogen atom, a lithium atom, a lower alkyl group, a group of formula

$$-\overset{\overset{O}{\|}}{C}-R_2 \qquad (A)$$

in which $R_2$ represents hydrogen, a lower alkyl group, a group $-OR_3$ in which $R_3$ represents hydrogen or a lower alkyl group or again $R_2$ represents a group $-N(R_4)_2$ in which $R_4$ represents a lower alkyl group,

a group of formula:

$$-\underset{\underset{R_5}{|}}{\overset{\overset{O}{\|}}{C}}-R_6 \qquad (B)$$

in which $R_5$ represents hydrogen or a lower alkyl group and $R_6$ represents phenyl or a lower alkyl group,

a silyl group of formula:

$$Sio(R_6)_3 \qquad (C)$$

in which $R_6$ has the same meaning as above, characterised in that

a) 1H-pyrrolo[3,2-c]pyridine is reacted, at room temperature and in a solvent, with an alkali metal hydroxide and in the presence of an interphase transfer catalyst, and then, at a temperature between room temperature and 40°C, with a halide of general formula:

$$R-Hal$$

in which R is defined as hereinbefore and Hal represents a chlorine, bromine or iodine atom, to obtain the N-protected 1H-pyrrolo[3,2-c]pyridine derivatives of formula III in which Z represents a hydrogen atom,

b) the N-protected 1H-pyrrolo[3,2-c]pyridine derivative obtained in paragraph a) is reacted in a solvent, at a temperature between −80°C and −20°C, with a lithiation agent, which is either a lithium amide or an alkyl lithium, in the presence of tetramethylethylenediamine to obtain the 2-lithio derivatives of formula III in which Z represents a lithium atom,

c) the 2-lithio-derivative obtained in paragraph b) is condensed, in a solvent and at a temperature between −80°C and room temperature, with a reagent capable of producing an electrophilic group selected from the group consisting of:

an alkyl halilde of general formula:

$$R_4-Hal$$

in which $R_4$ has the same meaning as above and Hal represents a halogen atom, to obtain the compounds of formula III in which Z represents a lower alkyl group,

an ester of general formula:

$$H-\overset{\overset{O}{\|}}{C}-OR_4$$

in which $R_4$ has the same meaning as above or an anhydride of general formula:

$$(R_4-\overset{\overset{O}{\|}}{C})_2O$$

in which $R_4$ has the same meaning as above, or carbonic anhydride or a halide of general formula:

$$Hal-\overset{\overset{O}{\|}}{C}-OR_4$$

in which Hal and $R_4$ have the same meaning as above, or a halide of general formula:

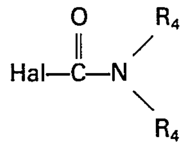

in which Hal and $R_4$ have the same meaning as above, to obtain the compounds of formula III in which Z represents a group of formula (A)

an aldehyde or a ketone of general formula:

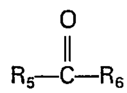

in which $R_5$ and $R_6$ have the same meaning as above, to obtain the compounds of formula III in which Z represents a group of formula (B)

a silyl halide of general formula:

$$Hal—Si(R_6)_3$$

in which Hal and $R_6$ have the same meaning as above to obtain the compounds of formula III in which Z represents a group (C).

3. Method according to claim 1 or 2, characterised in that R (the labile protecting group) is a methoxymethyl, benzyloxymethyl, benzenesulphonyl, p-toluenesulphonyl or tert.-butoxycarbonyl group.

4. Method according to claim 3, characterised in that the labile protecting group is a benzenesulphonyl or tert.-butoxycarbonyl group.

5. Method according to claim 1 or 2, characterised in that the lithium amide is lithium diisopropylamide.

6. Method according to claim 1 or 2, characterised in that 1 to 6 equivalents of tetramethylenediamine are used to each equivalent of N-protected 1H-pyrrolo[3,2-c]pyridine.

7. Method according to claim 1 or 2, characterised in that the solvent is an ether or an ether/hydrocarbon mixture.

8. Method according to Claim 7, characterised in that the ether is tetrahydrofuran and the ether/hydrocarbon mixture is a tetrahydrofuran/pentane mixture.

9. 1H-pyrrolo[3,2-c]pyridine derivatives of general formula:

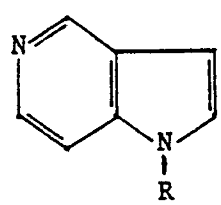

in which R represents a methoxymethyl, benzyloxymethyl, benzenesulphonyl, p-toluenesulphonyl or tert.-butoxycarbonyl group.

10. 1-Benzenesulphonyl-1H-pyrrolo[3,2-c]pyridine.

11. 1-Tert-butoxycarbonyl-1H-pyrrolo[3,2-c]pyridine.